# EUROPÄISCHE PATENTSCHRIFT

(11) **EP 3 999 142 B1**
(45) Veröffentlichungstag und Bekanntmachung des Hinweises auf die Patenterteilung: **18.01.2023**
(21) Anmeldenummer: 19801704.8
(22) Anmeldetag: 28.10.2019
(51) Int. Cl.: A61M 5/158

(54) **SCHUTZVORRICHTUNG FÜR DAS NADELROHR EINER SPRITZE**
PROTECTIVE DEVICE FOR THE NEEDLE TUBE OF A SYRINGE
DISPOSITIF DE PROTECTION POUR LE TUBE D'AIGUILLE D'UNE SERINGUE

(30) Priorität: 15.07.2019 DE 202019103876 U
(43) Veröffentlichungstag der Anmeldung: 25.05.2022
(73) Patentinhaber: Fischer, Stephan, 32120 Hiddenhausen (DE); Wilke, Tobias, 49477 Ibbenbüren (DE); Mohr, Bernd, 25355 Barmstedt (DE)
(72) Erfinder: Fischer, Stephan, 32120 Hiddenhausen (DE); Wilke, Tobias, 49477 Ibbenbüren (DE); Mohr, Bernd, 25355 Barmstedt (DE)
(74) Vertreter: Pellengahr, Maximilian Rudolf
(86) Internationale Anmeldenummer: PCT/EP2019/079385
(87) Internationale Veröffentlichungsnummer: WO 2021/008717

(56) Entgegenhaltungen:
- WO-A1-2018/151831
- WO-A1-2018/172853
- US-A- 5 885 249

## Beschreibung

### Einleitung

Die Erfindung betrifft eine Schutzvorrichtung für das Nadelrohr einer Spritze, umfassend ein an einem Trägerelement schwenkbar ausgebildetes Gehäuse für das Nadelrohr, welches in der Schwenkebene eine offene Gehäuseseite aufweist, sodass das Nadelrohr nach Injektionsgebrauch in das Gehäuse einschwenkbar ist und wobei gehäuseseitig eine Halteeinrichtung für das eingeschwenkte Nadelrohr vorgesehen ist.

### Stand der Technik

Es ist bekannt, dass die Nadel einer Injektionsspritze normalerweise ein mit einem hohlen Trägerelement verbundenes mit einer Spritze versehenes Metallrohr umfasst, das mit dem zylindrischen Körper einer Spritze verbunden ist. Die vorliegende Erfindung befasst sich hierbei mit dem Problem, das Metallrohr gegen Nadelstiche zu schützen, und hier insbesondere nachdem eine Injektion am Körper eines Patienten durchgeführt wurde, um so eine Injektion an Personen zu vermeiden, die mit der Nadel nach der Behandlung in Berührung kommen könnten. Wenn also eine zweite Person mit der Nadel gestochen würde, nachdem sie bei einer ersten Person verwandt wurde, kann es zu Übertragungen von Krankheiten kommen. Daher besteht mit dem Gebrauch dieser Nadel eine beträchtliche Gefahr, insbesondere für diejenigen, die normalerweise mit ihnen arbeiten, so insbesondere für Krankenhaus- und Arztpersonal. Die Entsorgung von benutzten Nadeln als Abfall birgt auch eine Gefahr für diejenigen, die mit den Abfallbehältern hantieren, oder sich diesen Behältern nähern. Aufgrund dieser Gegebenheiten ist es besonders wichtig, dass das Nadelrohr nach der Injektion geschützt ist, um auf diese Weise zu verhindern, dass es zu Krankheitsübertragungen kommen kann.

Um diesem Problem zu entgegnen, ist bereits aus der WO 2009/007718 A1 sowie dem deutschen Gebrauchsmuster DE 20 2018 107 052 eine Schutzvorrichtung für das Nadelrohr einer Spritze bekannt. Bei dieser aus dem Stand der Technik bekannten Schutzvorrichtung befindet sich das Nadelrohr der Spritze an einem Trägerelement, welches von diesem aufgenommen wird. An das Trägerelement bzw. auf das Nadelrohr wird die Auslassdüse der Spritze gesteckt. Somit ist die Schutzvorrichtung dann mit dem Spritzenkörper verbunden. Hierbei ist zum Schutz an dem Trägerelement ein schwenkbar gelagertes Gehäuse angeordnet, welches insbesondere an seiner Schwenkebene eine offene Gehäuseseite aufweist. Wird also die Spritze in Benutzung genommen, um eine Injektion vorzunehmen, wird das an dem Trägerelement schwenkbar gelagerte Gehäuse weggeschwenkt, sodass das Nadelrohr frei ist, um dann die Injektion vorzunehmen. Nach der Injektion wird das Gehäuse zurückgeschwenkt, wobei durch die offene Gehäuseseite das Nadelrohr wieder in das Gehäuse einschwenkt, sodass das Nadelrohr nach Injektionsgebrauch sich wieder in dem Gehäuse befindet. Damit nach Gebrauch ein unfreiwilliges Verschwenken des Gehäuseteils an dem Trägerelement vermieden wird, sind nach dem Stand der Technik Halteeinrichtungen in Form von Klemmen oder Rasten an den Gehäusewänden des schwenkbaren Gehäuses vorgesehen, die sich am Nadelrohr verkrallen, um so ein unfreiwilliges Herausschwenken des Nadelrohrs zu unterbinden. WO 2018/172853 A1 und US 5 885 249 A offenbaren teilweise die Merkmale, die in Anspruch 1 beansprucht sind.

Als nachteilig bei dieser nach dem Stand der Technik bekannten Schutzvorrichtung wird es angesehen, dass bei den bekannten Ausführungsformen ein sog. ungewollter Schwenkvorgang vorgenommen werden kann, sodass dann - ohne dass eine Injektion stattgefunden hat - hier das Nadelrohr freiliegt. Weiter wird es als nachteilig angesehen, dass die Halteeinrichtungen keinen sicheren Halt nach dem Injektionsverbrauch durch ein Rückverschwenken des Gehäuses gewährleisten.

Als Problem wird es auch angesehen, dass bereits schon bei nicht benutzten Spritzen es zu Problemen kommen kann, weil ein ungewollter Schwenkvorgang eingeleitet wird, der das Nadelrohr dann ungeschützt freilegt. Die Spritzen werden hierbei ineinander liegend in der Verpackung aufbewahrt. Wenn man nun aus dieser Verpackung eine oder mehrere Spritzen herausnimmt, ergibt sich das Problem, dass die Nadel selbsttätig hier aus dem Gehäuse, das mit einer Folie abgedeckt ist, herausspringen kann, weil die Umverpackung unsachgemäß geöffnet wird. Zudem ergibt sich das Problem, dass die bekannten Haltevorrichtungen hier nicht die gewünschte geschützte Lage des Nadelrohrs in dem Gehäuse vorhalten, wobei es immer noch dazu kommen kann, dass das Nadelrohr beim unsachgemäßen Entsorgen aus dem Gehäuse springen kann. Dies führt dazu, dass insbesondere die Haltevorrichtung nicht erkennen lässt, dass eine sichere Halterung der Nadel in dem Gehäuse vollzogen wurde.

Weiter wird es als nachteilig angesehen, dass die bekannten Ausführungsformen von Schutzvorrichtungen hinsichtlich ihrer Herstellungskosten sehr aufwändig sind, wobei insbesondere dabei auch der Effekt in Kauf genommen wird, dass bei einer günstigen Herstellung insbesondere wichtige Detailerfordernisse nicht berücksichtigt werden, weil dies die Kostenstruktur der Herstellung in Mitleidenschaft zieht.

### Aufgabe

Der Erfindung stellt sich somit das Problem, eine Schutzvorrichtung für ein Nadelrohr einer Spritze derart weiterzubilden, welche insbesondere die geschilderten Nachteile überwindet, wobei die Schutzvorrichtung dahingehend weitergebildet werden soll, dass diese wesentlich hygienischer in der sog. Parkstation gestaltet ist, unter der Voraussetzung, dass insbesondere die Herstellungskosten wesentlich verringert werden können.

### Lösung

Erfindungsgemäß wird dieses Problem mit den Merkmalen des Anspruchs 1 gelöst, vorteilhafte Ausgestaltungen der Erfindung ergeben sich aus den Unteransprüchen.

Die mit der Erfindung erreichten Vorteile bestehen nun darin, dass aufgrund der kostengünstigen Herstellung einer einstückigen Schutzvorrichtung für das Nadelrohr einer Spritze sämtliche Voraussetzungen für den sicheren Umgang mit dem Gegenstand bereitgestellt werden. Hierbei wird sichergestellt, dass ein ungewolltes Aufklappen des Gehäusebereichs vor Injektion unterbunden wird, da dies durch eine Sollbruchstelle, die hier angespritzt ist, verhindert wird. Weiterhin wird durch die erfindungsgemäßen Haltevorrichtungen sichergestellt, dass ein sich Selbstlösen des Nadelrohrs aus dem zurückgeschwenkten Gehäuse verhindert wird.

Gemäß der Erfindung wird daher vorgeschlagen, dass das Trägerelement und das schwenkbare Gehäuse neben der Verbindung eines Folienscharniers zur Bildung einer Schwenkachse zudem über eine Sollbruchstelle miteinander verbunden sind und wobei nach Injektionsgebrauch das Gehäuse über die zerstörte Sollbruchstelle zurückgeschwenkt werden kann. Aufgrund dieser Ausbildung wird erreicht, dass durch die einstückige Ausbildung der Schutzvorrichtung hier das Trägerelement mit dem schwenkbaren Gehäuse vor Gebrauch sich in einem festen Verbund befinden, wobei dadurch die Schwenkbarkeit nicht behindert wird, weil die sog. Sollbruchstelle hier erfordert, dass mit einem aufzubringenden Handdruck hier die Sollbruchstelle aufgerissen werden kann, sodass dann das Verschwenken erfolgen kann. Die Sollbruchstelle verhindert hier insbesondere, dass beim Entnehmen aus einer Packung es hier zu diesem sog. ungewollten Aufschwenken kommen kann. Auch befindet sich, nachdem die Sollbruchstelle zerstört worden ist, die Schutzvorrichtung immer noch in dem Zustand, dass eine sichere Aufbewahrung der Nadel nach Injektion in dem schwenkbaren Gehäuse vorgehalten werden kann, weil durch das Zurückschwenken des Gehäuses einerseits die Halteeinrichtung in dem Gehäuse greift, wobei andererseits durch das Überschwenken des Gehäuses dann das Nadelrohr mit der innen liegenden Verrasterung zusammenwirkt, sodass mit der Halteeinrichtung eine sichere Aufbewahrung nach Injektion des Nadelrohrs in dem schwenkbaren Gehäuse sichergestellt wird.

In Weiterbildung der Erfindung besteht die Sollbruchstelle aus gespritzten flexibel gehaltenen Stegelementen. Hierbei sind die Stegelemente auf den Randbereichen von Trägerelement und Gehäuserand verbindend angeordnet, wobei diese verteilt über den Randbereich von Trägerelement und Gehäuse angespritzt sind.

Gemäß einer vorteilhaften Ausgestaltung ist nach Injektionsgebrauch das Gehäuse über die Sollbruchstelle in eine Raststellung zurückschwenkbar, wobei das Nadelrohr mit einer im Gehäuse vorgesehenen Halteeinrichtung in Wirkverbindung bringbar ist. Bei dieser Ausführungsform wird das Gehäuse über einen Schwenkpunkt hinaus verschwenkt, sodass dann in der verrasteten Endstellung Trägerelement und Gehäuse in einer leichten Schrägstellung zueinander stehen. Dies hat insbesondere den Vorteil, dass in der Schrägstellung sichergestellt wird, dass die Halteeinrichtung in dem Gehäuse für das Nadelrohr greift.

Gemäß der Erfindung wird hierzu eine Halteeinrichtung vorgeschlagen, die im Gehäuse aus zwei an der Gehäusewand angeformten Zungen besteht, die in Einschwenkrichtung des Nadelrohrs harpunenartig ausgerichtet sind. Um der Nadel beim Einschwenkvorgang entsprechend eine Zwangsführung zu geben, sodass die Nadel entweder hinter der einen oder der anderen Zunge springt, ist zwischen den Zungen ein in Dreiecksform ausgebildetes Zapfenelement vorgesehen, welches das einschwenkte Nadelrohr unter die eine oder die andere Zunge gezwungenermaßen drückt.

In Weiterbildung der Schutzvorrichtung als gespritztes einstückiges Bauteil besteht hierbei die Halteeinrichtung aus einer eindrückbaren Hülse, die über sägezahnartige Sollbruchstellen mit dem Gehäuse verbunden ist. Die Sollbruchstellen sind an die Gehäusestirnseite mittels überstehender Stegstützen angeformt, wobei die Stegstützen außen an der Gehäusewand angeformt sind. Aufgrund dieser Ausbildung wird hier eine Halteeinrichtung in Form einer eindrückbaren Hülse bereitgestellt, die mit dem Gehäuseteil unmittelbar mit angespritzt werden kann, was insbesondere hier auch die Herstellungskosten wesentlich vergünstigt.

Somit ist festzuhalten, dass die Schutzvorrichtung mit Trägerelementen, Nadelgehäuse, Sollbruchstelle sowie den Halteeinrichtungen einstückig aus einem Spritzgussformteil gefertigt ist.

Nach einer vorteilhaften Weiterbildung der Erfindung kann optional mit dem am Trägerelement schwenkbar gelagerten Gehäuse eine Schutzhülse zusammenwirken, mittels der nach Injektionsgebrauch die in der Schwenkebene liegende offene Gehäuseseite verschlossen werden kann. Die Schutzhülse ermöglicht es die Schutzvorrichtung, in der das Nadelrohr geklemmt und verhakt liegt zusätzlich hermetisch zu verschließen. Hierzu weist die Schutzhülse eine in der Wandung angeordnete Öffnung auf, die mit der offenen am Gehäuse liegenden Seite deckungsgleich ist. Somit behindert die Schutzhülse nicht das Herausschwenken der Nadel, weil die vorgehaltene Nadel durch die Öffnung der Schutzhülse hindurchschwenken kann. Die Schutzhülse ist drehbar an dem Gehäuse gelagert, so dass sie von einer nach Injektionsgebrauch offenen Stellung in eine das Gehäuse verschließende Stellung gedreht werden kann.

Die Schutzhülse ist hierbei mit ihrem unteren Gehäuserand am Randbereich des Gehäuses mittels Formschluss verbunden und drehbar gelagert. Um ein ungewolltes Verdrehen der Schutzhülse nach Injektionsgebrauch am Gehäuse zu unterbinden, ist am Randbereich des Gehäuses ein Rastelement angeformt, welches nach Verdrehen der Schutzhülse um 180° am Gehäuse mit einer in der Wand der Schutzhülse angeordneten Durchdringung zusammenwirkt, die die Raststellung der Schutzhülse am Gehäuse bewirkt. dass die Schutzhülse an ihrem oberen Deckbereich ein Griffelement aufweist, an dessen Unterseite ein Ringelement angeformt ist mit einer eingeformten 180° Steuerkurve, die mit einem auf dem Deckbereich des Gehäuses angeformten Keil zusammenwirkt, der ein Zurückdrehen der Schutzhülle nach Verrasterung der Steuerkurve mit dem Keil unterbindet.

Nach einer besonders vorteilhaften Weiterbildung weist die Schutzhülse an ihrem oberen Deckbereich ein Griffelement auf. An der Unterseite des Griffelementes ist ein Ringelement angeformt, wobei in dem Ringelelement eine 180° gebogene Steuerkurve eingeformt ist. Die eingeformte Steuerkurve wirkt hierbei mit einem auf dem Deckbereich des Gehäuses angeformten Keil zusammen, der beim Überfahren der Steuerkurve im Endbereich des Bogens einrastet. Somit wird ein Zurückdrehen der Schutzhülle nach Verrasterung der Steuerkurve mit dem Keil unterbunden. Zur Festlegung des Griffelementes an der Schutzhülle weist das Ringelement eine in der Rotationachse liegende kreisförmige Durchdringung auf, durch die eine durch die kreisförmige Durchdringung greifende Spannrasterung am Deckbereich des Gehäuses greift.

### Ausführungsbeispiele

Die Erfindung ist nachstehend anhand eines Ausführungsbeispiels, das in den Figuren dargestellt ist, näher erläutert. Es zeigt:
- Fig. 1:: Eine perspektivische Darstellung einer Schutzvorrichtung gemäß einer ersten Ausführungsform,
- Fig. 2:: eine weitere perspektivische Darstellung gemäß einer weiteren Ausführungsform,
- Fig. 3:: eine Detailansicht insbesondere der erfindungsgemäßen Sollbruchstelle zwischen Trägerelement und schwenkbarem Gehäuse,
- Fig. 4:: eine weitere perspektivische Darstellung gemäß der Figur 3 in einer anderen Ansicht,
- Fig. 5:: eine weitere Darstellung gemäß den Figuren 3 und 4 im eingerasteten Zustand in Schrägstellung des Gehäuses zum Trägerelement,
- Fig. 6:: eine Detailansicht der Halteeinrichtung einer ersten Ausführungsform
- Fig. 7:: eine weitere Darstellung der Halteeinrichtung in Detailansicht gemäß der Figur 6 in der sog. eingerasteten Stellung,
- Fig. 8:: eine weitere Seitenansicht der Halteeinrichtung gemäß der zweiten Ausführungsform, und zwar gemäß der Figur 2,
- Fig. 9:: eine perspektivische Darstellung der Schutzvorrichtung mit Schutzhülse in der Offenstellung,
- Fig. 10:: eine teilgeschnittene Frontansicht gemäß der Figur 9,
- Fig. 11:: eine weitere perspektivische Darstellung der Schutzvorrichtung mit Schutzhülse in der geschlossenen Stellung,
- Fig. 12:: eine perspektivische Darstellung eines Griffelementes für die Schutzhülle in Wirkverbindung mit dem Gehäuse in gestrichelter Darstellung und
- Fig. 13:: eine weitere perspektivische Darstellung des Griffelementes in Unteransicht.

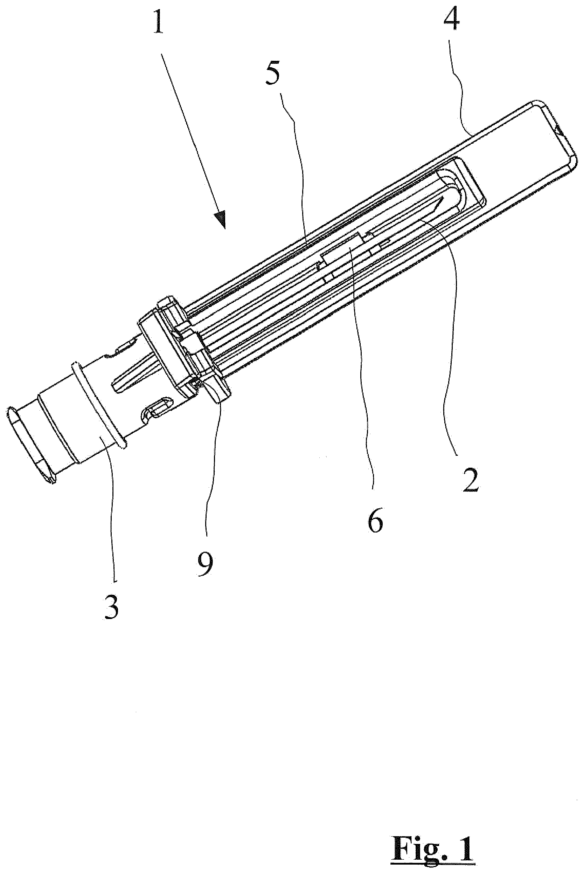

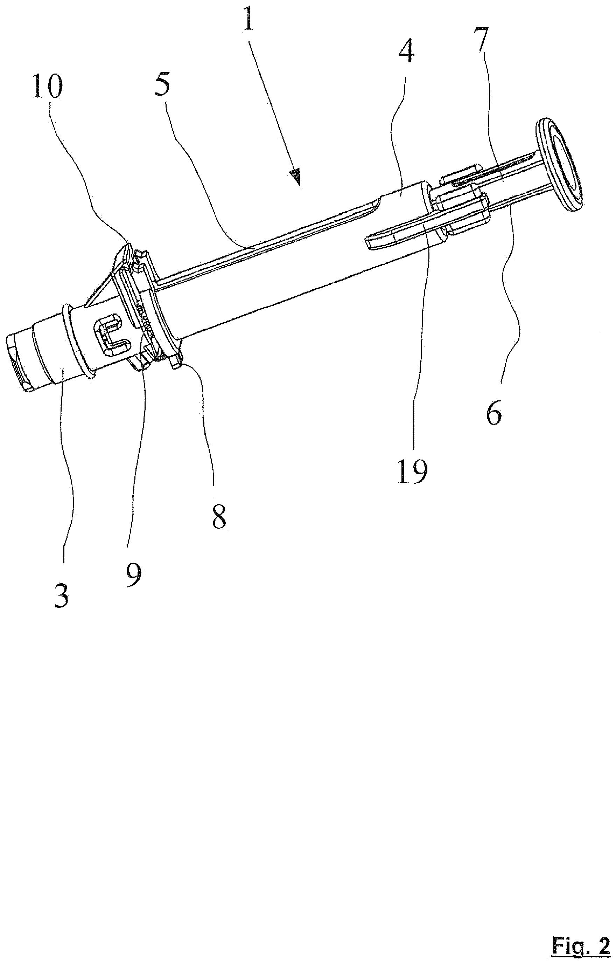

Die Figuren 1 und 2 zeigen jeweils in perspektivischen Darstellungen eine Schutzvorrichtung 1 für ein Nadelrohr 2 einer nicht näher dargestellten Spritze in zwei unterschiedlichen Ausführungsformen. Die Schutzvorrichtung 1 umfasst hierbei ein an einem Trägerelement 3 schwenkbar ausgebildetes Gehäuse 4 für das Nadelrohr 2, welches in der Schwenkebene des Gehäuses 4 eine offene Gehäuseseite 5 aufweist. Aufgrund der offenen Gehäuseseite 5 besteht die Möglichkeit, dass das Nadelrohr 2 nach Injektionsgebrauch in das Gehäuse 4 eingeschwenkt werden kann, sodass es dann geschützt ist.

Um insbesondere das Nadelrohr 2 vor unsachgemäßem Gebrauch nach der Injektion zu schützen, ist gehäuseseitig eine Haltereinrichtung 6 für das eingeschwenkte Nadelrohr 2 vorgesehen, wobei die Figur 1 eine Halteeinrichtung 6 innerhalb des Gehäuses 4 zeigt, die hier als Klemmeinrichtung ausgebildet ist, wobei die Figur 2 die Halteeinrichtung 6 darstellt, bei der zum Schutz das Nadelrohr 2 von einer Hülse 7 überstülpt wird.

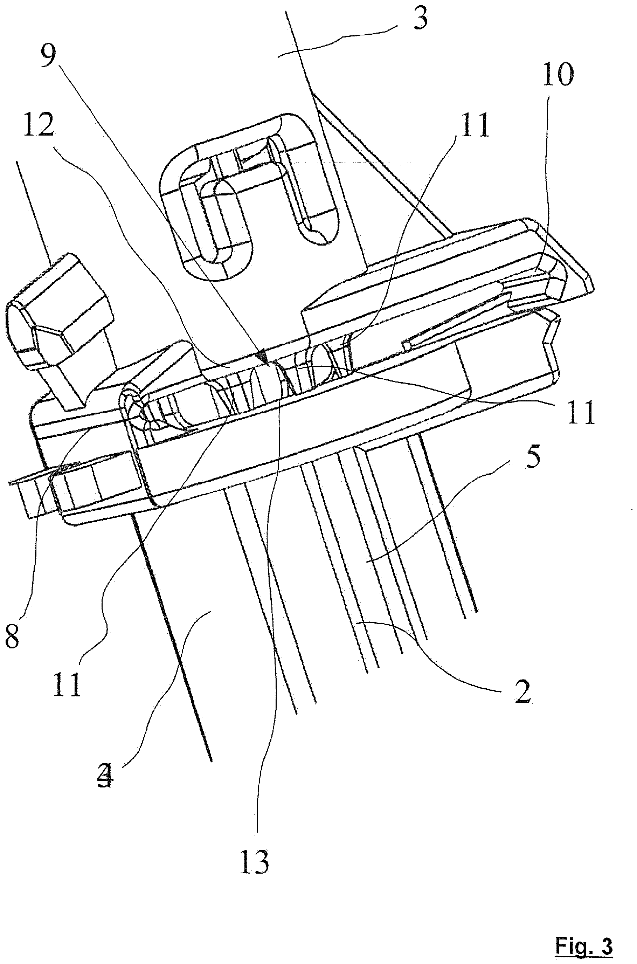

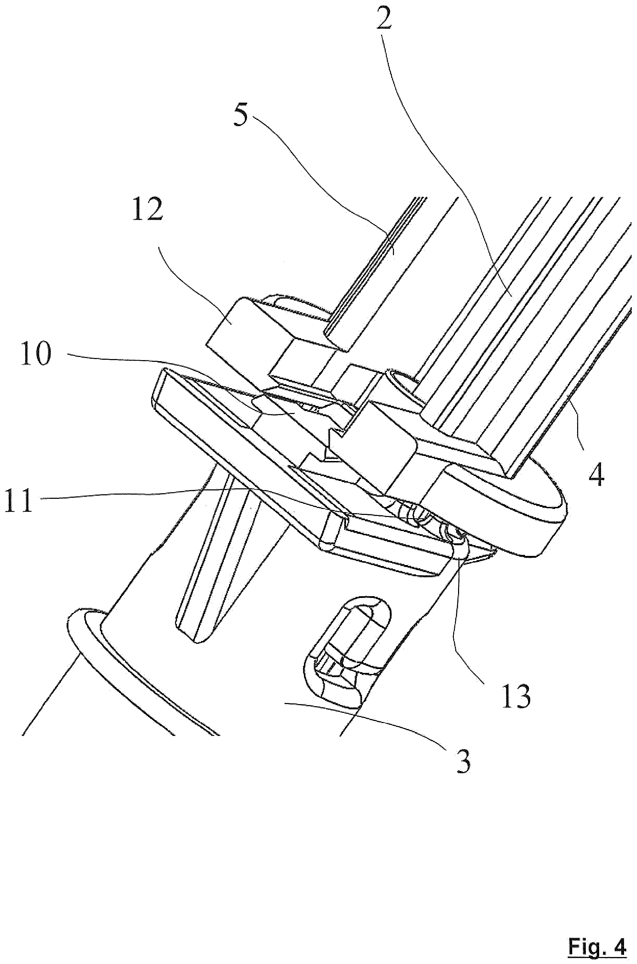

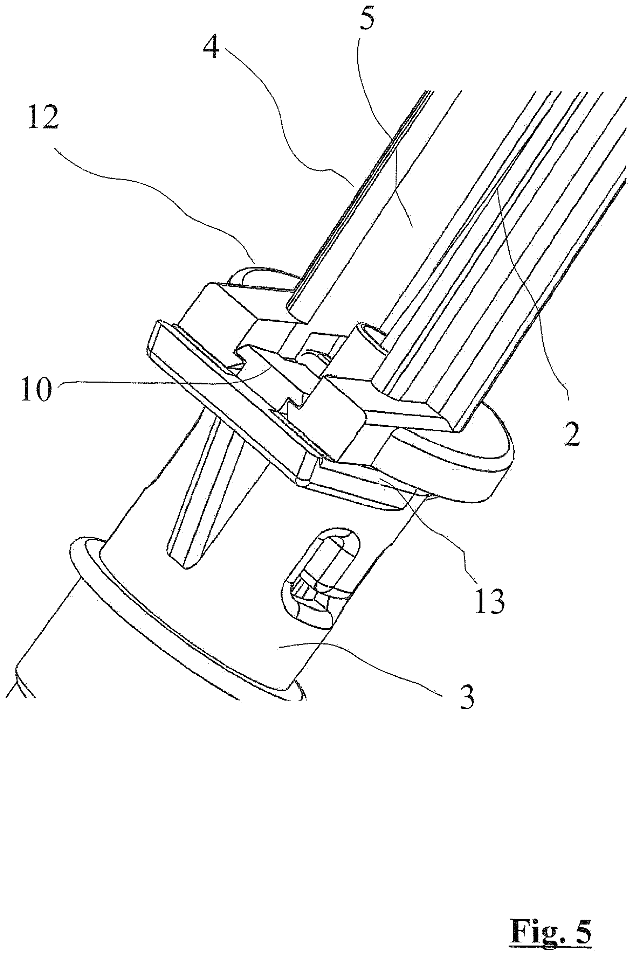

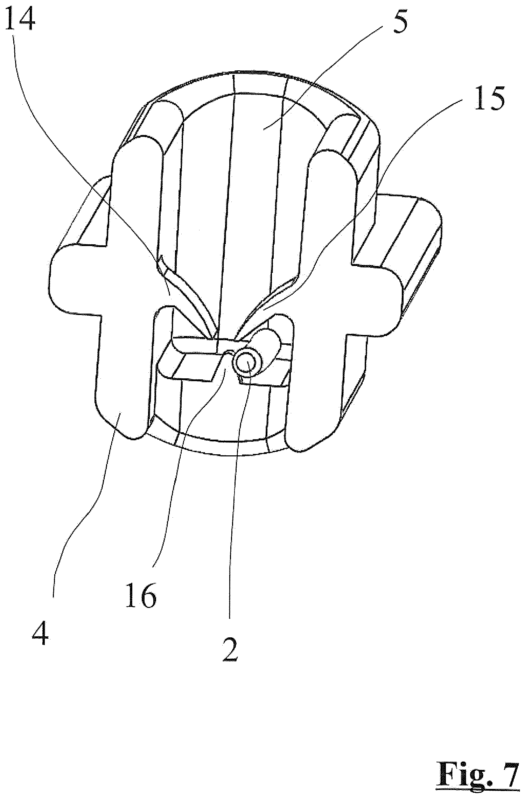

Gemäß der erfindungsgemäßen Schutzvorrichtung 1 verfügt das Trägerelement 3 und das schwenkbare Gehäuse 4 neben der Verbindung eines Folienscharniers 8, näher zu erkennen in den Figuren 3 und 4, zur Bildung einer Schwenkachse zudem über eine Sollbruchstelle 9, über die das Trägerelement 3 und das Gehäuse 4 hierbei verbunden sind. Wird die Sollbruchstelle 9 zerstört, besteht bei der erfindungsgemäßen Ausführungsform die Möglichkeit, dass nach Injektionsgebrauch das Gehäuse 4 über die zerstörte Sollbruchstelle 9 zurückgeschwenkt werden kann, wie dies in der Figur 5 dargestellt ist, wobei dann eine Rasteinrichtung 10, dargestellt in der Figur 5, in Wirkung tritt. In dieser Lage der Figur 5 befindet sich das Gehäuse 4 gegenüber dem Trägerelement 3 in einer leicht schräg gestellten Lage, sodass insbesondere die Halteeinrichtung 6, so wie sie in der Figur 7 dargestellt ist, ebenfalls in Wirkverbindung tritt. Bei dieser Lage befindet sich das Gehäuse 4 in der verrasteten Lage, dass es eine leichte Schrägstellung über das Folienscharnier 8 einnimmt, wobei dann die Rasten der Rasteinrichtung 10 wirken.

Wie insbesondere aus den Figuren 3 und 4 näher zu erkennen ist, besteht die Sollbruchstelle 9 aus gespritzten flexibel gehaltenen Stegelementen 11, die auf den Randbereichen 12 und 13 von Trägerelement 3 und Gehäuse 4 angeordnet sind. Hierbei sind die Stegelemente 11 verteilt über den Randbereichen 12 und 13 angeordnet, wie dies in der Zusammenschau der Figuren 3 und 4, aber auch angedeutet in der Figur 2, erkennbar ist. Wie bereits ausgeführt, ist nach Injektionsgebrauch das Gehäuse 4 über die Sollbruchstelle 9 in eine Raststellung, dargestellt in den Figuren 4 und 5, zurückschwenkbar, wobei das Nadelrohr 2 mit einer im Gehäuse 4 vorgesehenen Halteeinrichtung 6 in Wirkverbindung bringbar ist.

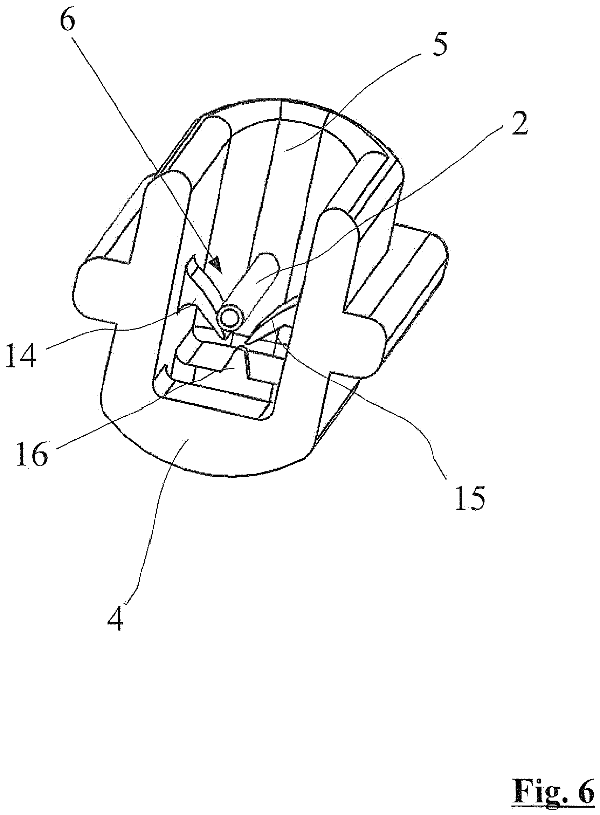

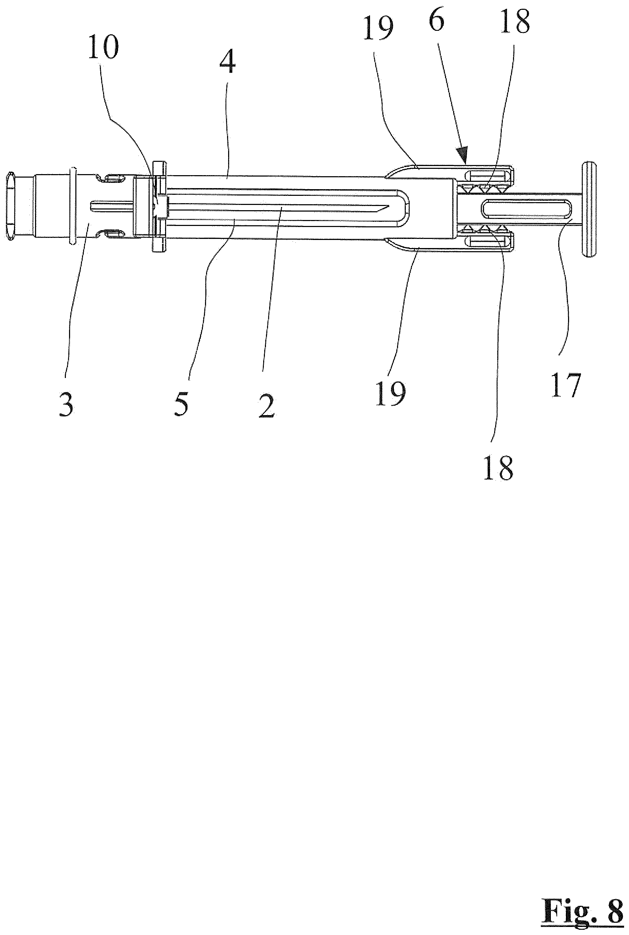

Die unterschiedlichen Halteeinrichtungen 6 sind hier in den Figuren 6 und 7 sowie in den Figuren 1 und 8 näher gezeigt. Dabei zeigen die Figuren 6 und 7 auch i. V. m. der Figur 1 eine Halteeinrichtung 6, die im Gehäuse 4 aus zwei an der Gehäusewand angeformten Zungen 14 und 15 besteht, wie diese insbesondere in den Figuren 6 und 7 näher zu erkennen sind. Dabei sind die Zungen 14 und 15 harpunenartig derart ausgerichtet, dass sie in Einschwenkrichtung des Nadelrohrs 2 nach Durchfahren des Nadelrohrs 2 dieses hintergreifen. Um den Hintergreifungsprozess zu begünstigen, ist zwischen den Zungen 14 und 15 ein in Dreiecksform ausgebildetes Zapfenelement 16 vorgesehen, welches das eingeschwenkte Nadelrohr 2 unter die eine Zunge 14 oder die andere Zunge 15 drückt. Es versteht sich nun von selbst, dass wenn das Nadelrohr 2 einschwenkt und die sog. Raststellung gemäß der Figur 5 erzeugt wird, sich dann die Stellung des Nadelrohrs 2 gemäß der Figur 7 einstellt, wobei das Nadelrohr 2 beim Einschwenken entweder nach links oder rechts gedrückt wird und so dann die gesicherte Position des Zurückhaltens erfährt.

Gemäß einer weiteren Ausführungsform trifft die Halteeinrichtung 6, insbesondere die Darstellung der Figur 8 i. V. m. der Figur 2, wo die Halteeinrichtung 6 aus einer eindrückbaren Hülse 17 besteht, die über sägezahnartige Sollbruchstellen 18 mit dem Gehäuse 4 verbunden ist. Wie zu erkennen ist, sind die Sollbruchstellen 18 an die Gehäuseseite mit überstehenden Stegstützen 19 angeformt. Die Stegstützen 19 sind hierbei an der Gehäusewand von außen angeformt, wie dies insbesondere schön aus der Figur 2, aber auch aus der Figur 8 in der geschnittenen Darstellung zu erkennen ist. Bei beiden Ausführungsformen der Halteeinrichtung 6 ist Folgendes berücksichtigt, dass insbesondere diese Halteeinrichtungen 6, wie sie in den Figuren 6, 7 aber auch 8 beschrieben und dargestellt sind, im einstückigen Spritzgussverfahren mitgespritzt werden, sodass neben dem Gehäuse 4 und dem Trägerelement 3 auch die Halteeinrichtung 6 hier mitgespritzt und geformt wird.

Entsprechendes gilt somit für die gesamte Schutzvorrichtung 1, die hier als einstückiges Teil derart gespritzt werden kann, dass diese neben der Sollbruchstellen 9, 18, dem Folienscharnier 8 den Rasteinrichtungen 10 sowie den beiden unterschiedlichen Halteeinrichtungen 6 in einem Gang bzw. in einem Werkzeug gespritzt werden können.

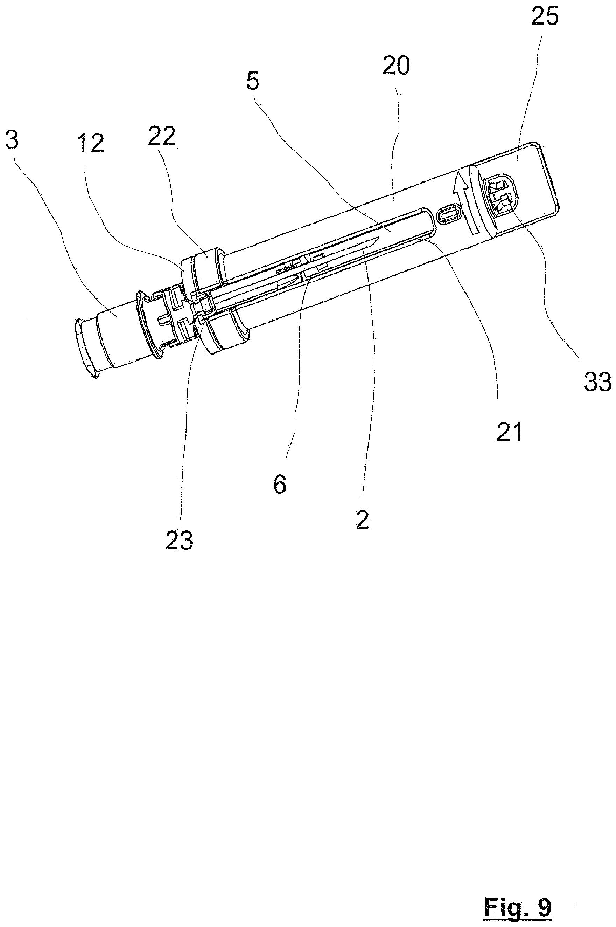

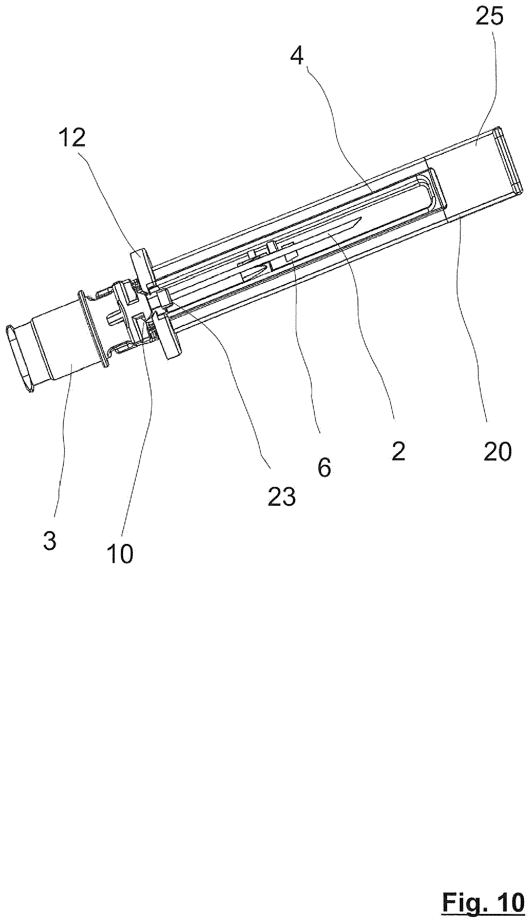

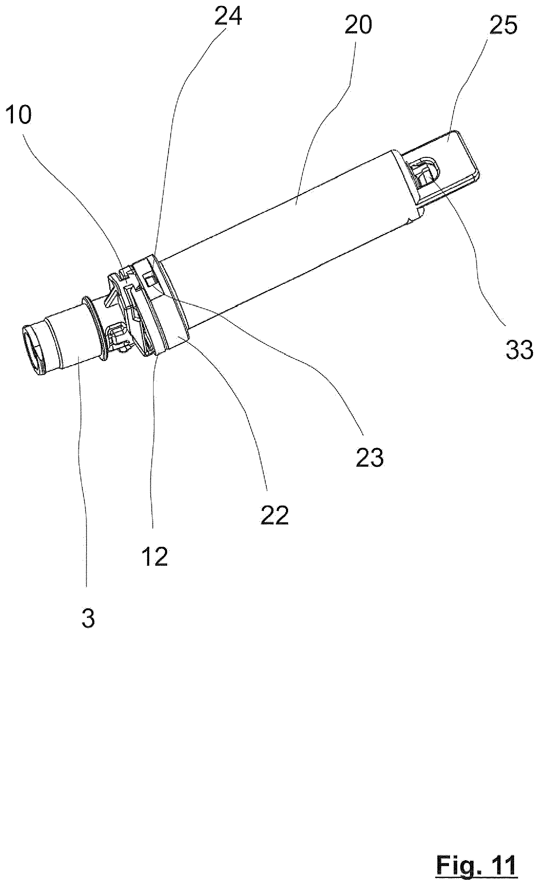

Nach einer vorteilhaften Weiterbildung der Erfindung, dargestellt in den Figuren 9, 10 und 11, kann optional mit dem am Trägerelement 3 schwenkbar gelagerten Gehäuse 4 eine Schutzhülse 19 zusammenwirken, mittels der nach Injektionsgebrauch die in der Schwenkebene liegende offene Gehäuseseite 5 verschlossen werden kann. Die Schutzhülse 19 ermöglicht es die Schutzvorrichtung 1, in der das Nadelrohr 2 geklemmt und verhakt liegt, zusätzlich hermetisch zu verschließen. Hierzu weist die Schutzhülse 20 eine in der Wandung angeordnete Öffnung 21 auf, die mit der offenen am Gehäuse liegenden Seite 5 deckungsgleich ist. Somit behindert die Schutzhülse 20 nicht das Herausschwenken des Nadelrohres 2, weil das vorgehaltene Nadelrohr 2 durch die Öffnung 21 der Schutzhülse 20 hindurchschwenken kann. Die Schutzhülse 20 ist drehbar an dem Gehäuse 4 gelagert, so dass sie von einer nach Injektionsgebrauch offenen Stellung, dargestellt in der Figur 9, in eine das Gehäuse 4 verschließende Stellung, dargestellt in der Figur 11, gedreht werden kann.

Die Schutzhülse 20 ist hierbei mit ihrem unteren Gehäuserand 22 am Randbereich 12 des Gehäuses 4 mittels Formschluss verbunden und drehbar gelagert. Der Formschluss kann hier beispielsweise durch eine Klippverbindung erfolgen. Um ein ungewolltes Verdrehen der Schutzhülse 20 nach Injektionsgebrauch am Gehäuse 4 zu unterbinden, ist am Randbereich 12 des Gehäuses 4 ein Rastelement 23 angeformt, welches nach Verdrehen der Schutzhülse 20 um 180°, gezeigt in der Figur 11, am Gehäuse 4 mit einer in der Wand der Schutzhülse 20 angeordneten Durchdringung 24 zusammenwirkt, die die Raststellung der Schutzhülse 20 am Gehäuse 4 bewirkt.

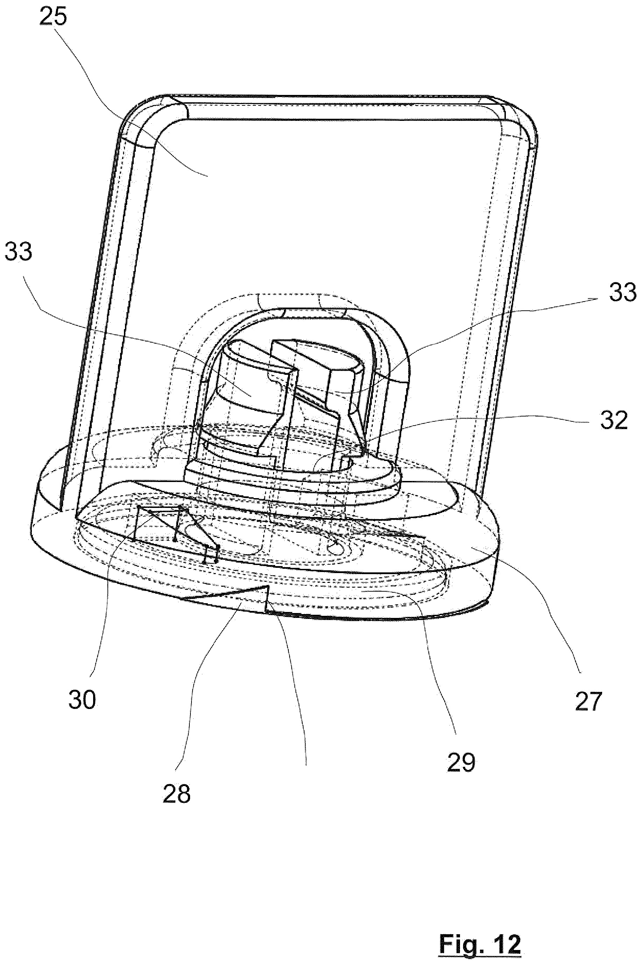

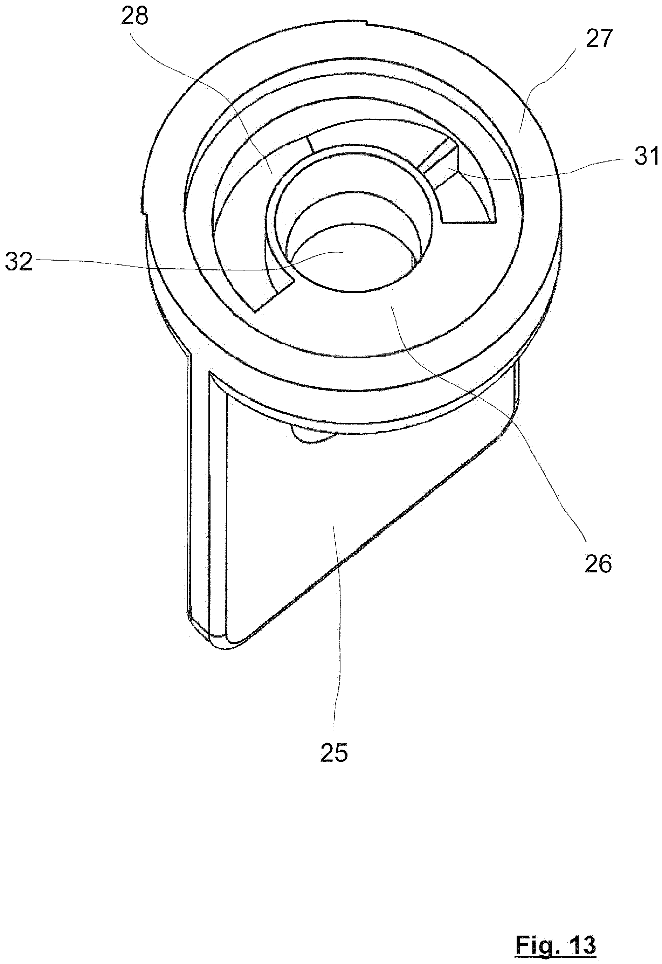

Nach einer besonders vorteilhaften Weiterbildung, dargestellt in den Figuren 12 und 13 weist die Schutzhülse 20 an ihrem oberen Deckbereich ein Griffelement 25 auf. An der Unterseite 26 des Griffelementes 25 ist ein Ringelement 27 angeformt, wobei in dem Ringelelement 27 eine 180° gebogene Steuerkurve 28 eingeformt ist. Die eingeformte Steuerkurve 28 wirkt hierbei mit einem auf dem Deckbereich 29 des Gehäuses 4 angeformten Keil 30 zusammen, der beim Überfahren der Steuerkurve 28 im Endbereich des Bogens an der Stufe 31 einrastet. Somit wird ein Zurückdrehen der Schutzhülle 20 nach Verrasterung der Steuerkurve 28 mit dem Keil 30 unterbunden.

Zur Festlegung des Griffelementes 25 an der Schutzhülle 20 weist das Ringelement 27 eine in der Rotationachse liegende kreisförmige Durchdringung 32 auf, durch die Spannzungen 33 greifen, die am Deckbereich des Gehäuses 4 angeformt sind. Die Spannzungen 33 verrasten sich im eingesteckten Zustand des Griffelementes 25 dann an dem Rand der kreisförmigen Durchdringung 32, wobei dann auch entsprechend wie beschrieben die Steuerkurve 28 mit dem Keil 30 in Wirkverbindung tritt.

### Bezugszeichenliste

- 1: Schutzvorrichtung
- 2: Nadelrohr
- 3: Trägerelement
- 4: Gehäuse
- 5: offene Gehäuseseite
- 6: Halteeinrichtung
- 7: Hülse
- 8: Folienscharnier
- 9: Sollbruchstelle
- 10: Rasteinrichtung
- 11: Stegelemente
- 12: Randbereich
- 13: Randbereich
- 14: Zunge
- 15: Zunge
- 16: Zapfenelement
- 17: Hülse
- 18: Sollbruchstellen Sägezahn
- 19: Stegstützen
- 20: Schutzhülse
- 21: Öffnung in der Wand der Schutzhülse
- 22: Gehäuserand unten Schutzhülle
- 23: Rastelement
- 24: Durchdringung
- 25: Griffelement
- 26: Unterseite Griffelement
- 27: Ringelement
- 28: Steuerkurve
- 29: Deckbereiche Gehäuse
- 30: Keil
- 31: Stufe
- 32: Durchdringung Ringelement
- 33: Spannzungen

## Patentansprüche

1. Schutzvorrichtung (1) für das Nadelrohr (2) einer Spritze, umfassend
ein an einem Trägerelement (3) schwenkbar ausgebildetes Gehäuse (4), welches in der Schwenkebene eine offene Gehäuseseite (5) aufweist, sodass das Nadelrohr (2) nach injektionsgebrauch in das Gehäuse (4) einschwenkbar ist,
wobei das Trägerelement (3) und das schwenkbare Gehäuse (4) neben der Verbindung eines Folienscharniers (8) zur Bildung einer Schwenkachse zudem über eine Sollbruchstelle (9) miteinander verbunden sind,
**dadurch gekennzeichnet, dass**
gehäuseseitig eine Halteeinrichtung (6) für das eingeschwenkte Nadelrohr (2) vorgesehen ist,
wobei nach Injektionsgebrauch das Gehäuse (4) über die zerstörte Sollbruchstelle (9) in eine Raststellung zurückschwenkbar ist,
wobei das Nadelrohr (2) mit der Halteeinrichtung (6) in Wirkverbindung bringbar ist, die im Gehäuse (4) vorgesehen ist.

2. Schutzvorrichtung nach Anspruch 1,
**dadurch gekennzeichnet,**
**dass** die Sollbruchstelle (9) aus gespritzten, flexibel gehaltenen Stegelementen (11) besteht.

3. Schutzvorrichtung nach den Ansprüchen 1 und 2,
**dadurch gekennzeichnet,**
**dass** die Stegelemente (11) auf den Randbereichen (12, 13) von Trägerelement (3) und Gehäuse (4) angeordnet sind.

4. Schutzvorrichtung nach den Ansprüchen 1 bis 3,
**dadurch gekennzeichnet,**
**dass** die Stegelemente (11) verteilt über den Randbereichen (12, 13) von Trägerelement (3) und Gehäuse (4) angeordnet sind.

5. Schutzvorrichtung nach den Ansprüchen 1 bis 4
**dadurch gekennzeichnet,**
**dass** die Halteeinrichtung (6) im Gehäuse (4) aus zwei an der Gehäusewand angeformten Zungen (14, 15) besteht, die in Einschwenkrichtung des Nadelrohres (2) harpunenartig ausgerichtet sind.

6. Schutzvorrichtung nach den Ansprüchen 1 bis 5,
**dadurch gekennzeichnet,**
**dass** zwischen den Zungen (14, 15) ein in Dreiecksform ausgebildetes Zapfenelement (16) vorgesehen ist, welches das eingeschwenkte Nadelrohr (2) unter die eine oder die andere Zunge (14), (15) drückt.

7. Schutzvorrichtung nach den Ansprüchen 1 bis 6,
**dadurch gekennzeichnet,**
**dass** die Halteeinrichtung (6) aus einer eindrückbaren Hülse (17) besteht, die über sägezahnartige Sollbruchstellen (18) mit dem Gehäuse (4) verbunden ist.

8. Schutzvorrichtung nach Anspruch 7,
**dadurch gekennzeichnet,**
**dass** die Sollbruchstellen (18) an die Gehäusestirnseite überstehenden Stegstützen (19) angeformt sind.

9. Schutzvorrichtung nach Anspruch 8,
**dadurch gekennzeichnet,**
**dass** die Stegstützen (19) an der Gehäusewand angeformt sind.

10. Schutzvorrichtung nach einem oder mehreren der Ansprüche 1 bis 9,
**dadurch gekennzeichnet,**
**dass** die Schutzvorrichtung (1) mit Trägerelement (3), Nadelgehäuse (4), Sollbuchstelle (9), sowie der Halteeinrichtung (6) einstückig aus einem Spritzgussformteil gefertigt ist.

11. Schutzvorrichtung nach den Ansprüchen 1 bis 6,
**dadurch gekennzeichnet,**
**dass** mit dem am Trägerelement (3) schwenkbar gelagerten Gehäuse (4) optional eine Schutzhülse (20) zusammenwirkt, mittels der nach Injektionsgebrauch die in der Schwenkebene liegende offene Gehäuseseite (5) verschließbar ist.

12. Schutzvorrichtung nach Anspruch 11,
**dadurch gekennzeichnet,**
**dass** die Schutzhülse (20) eine in der Wandung angeordnete Öffnung (21) aufweist, die mit der offenen am Gehäuse liegenden Seite (5) deckungsgleich ist.

13. Schutzvorrichtung nach den Ansprüchen 11 und 12,
**dadurch gekennzeichnet,**
**dass** die Schutzhülse (20) drehbar an dem Gehäuse (4) gelagert ist, so dass die Schutzhülse (20) von einer nach Injektionsgebrauch offenen Stellung in eine das Gehäuse (4) verschließende Stellung drehbar ist.

14. Schutzvorrichtung nach den Ansprüchen 11 bis 13,
**dadurch gekennzeichnet,**
**dass** die Schutzhülse (20) mit ihrem unteren Gehäuserand (22) am Randbereich (12) des Gehäuses (4) drehbar gelagert ist, und wobei am Randbereich (12) des Gehäuses (4) ein Rastelement (23) angeformt, das nach Verdrehen der Schutzhülse (20) um 180° am Gehäuse (4) mit einer in der Wand der Schutzhülse (20) angeordneten Durchdringung (24) in die Raststellung tritt.

15. Schutzvorrichtung nach den Ansprüchen 11 bis 14,
**dadurch gekennzeichnet,**
**dass** die Schutzhülse (20) an ihrem oberen Deckbereich ein Griffelement (25) aufweist, an dessen Unterseite (26) ein Ringelement (27) mit einer eingeformten 180° Steuerkurve (28) eingeformt ist, die mit einem auf dem Deckbereich (29) des Gehäuses (4) angeformten Keil (30) zusammenwirkt, der ein Zurückdrehen der Schutzhülle (20) nach Verrasterung der Steuerkurve (28) mit dem Keil (30) unterbindet.

16. Schutzvorrichtung nach den Ansprüchen 11 bis 14,
**dadurch gekennzeichnet,**
**dass** das Griffelement (25) mittels durch das Ringelement (27) greifende Spannzungen (33) am Deckbereich (29) des Gehäuses (4) festlegbar ist.

## Claims

1. A protective device (1) for the needle tube (2) of a syringe, comprising
a housing (4) which is designed to be pivotable on a carrier element (3), which housing has an open housing side (5) in the pivot plane, so that the needle tube (2) is able to be pivoted into the housing (4) after being used for injection,
wherein the carrier element (3) and the pivotable housing (4), in addition to the connection of a film hinge (8) for the formation of a pivot axis are additionally connected to one another via a predetermined breaking point (9),
**characterized in that**
on the housing side, a holding device (6) is provided for the pivoted-in needle tube (2),
wherein after being used for injection, the housing (4) is able to be pivoted back into a detent position via the destroyed predetermined breaking point (9),
wherein the needle tube (2) is able to be brought into operative connection with the holding device (6), which is provided in the housing (4).

2. The protective device according to Claim 1,
**characterized in that**
the predetermined breaking point (9) consists of injected, flexibly held web elements (11).

3. The protective device according to Claims 1 and 2,
**characterized in that**
the web elements (11) are arranged on the edge regions (12, 13) of carrier element (3) and housing (4).

4. The protective device according to Claims 1 to 3,
**characterized in that**
the web elements (11) are arranged distributed over the edge regions (12, 13) of carrier element (3) and housing (4).

5. The protective device according to Claims 1 to 4,
**characterized in that**
the holding device (6) in the housing (4) consists of two tongues (14, 15) formed on the housing wall, which are aligned in a harpoon-like manner in the pivoting-in direction of the needle tube (2).

6. The protective device according to Claims 1 to 5,
**characterized in that**
between the tongues (14, 15) a pin element (16), configured in triangular shape, is provided, which presses the pivoted-in needle tube (2) under the one or the other tongue (14), (15).

7. The protective device according to Claims 1 to 6,
**characterized in that**
the holding device (6) consists of a sleeve (17) which is able to be pressed in, which is connected to the housing (4) via sawtooth-like predetermined breaking points (18).

8. The protective device according to Claim 7,
**characterized in that**
the predetermined breaking points (18) are formed at web supports (19) projecting over the housing face side.

9. The protective device according to Claim 8,
**characterized in that**
the web supports (19) are formed on the housing wall.

10. The protective device according to one or more of Claims 1 to 9,
**characterized in that**
the protective device (1) with carrier element (3), needle housing (4), predetermined breaking point (9), and the holding device (6), is formed in one piece from an injection moulded part.

11. The protective device according to Claims 1 to 6,
**characterized in that**
optionally a protective sleeve (20) cooperates with the housing (4) mounted pivotably on the carrier element (3), by means of which protective sleeve, after use for injection, the open housing side (5) lying in the pivot plane is able to be closed.

12. The protective device according to Claim 11,
**characterized in that**
the protective sleeve (20) has an opening (21) arranged in the wall, which opening is congruent with the open side (5) lying on the housing.

13. The protective device according to Claims 11 and 12,
**characterized in that**
the protective sleeve (20) is rotatably mounted on the housing (4), so that the protective sleeve (20) is rotatable from an open position after use for injection into the position closing the housing (4).

14. The protective device according to Claims 11 to 13,
**characterized in that**
the protective sleeve (20) is rotatably mounted with its lower housing edge (22) on the edge region (12) of the housing (4), and wherein at the edge region (12) of the housing (4) a detent element (23) is formed on, which, after twisting of the protective sleeve (20) through 180° on the housing (4), enters into the detent position with a penetration (24) arranged in the wall of the protective sleeve (20).

15. The protective device according to Claims 11 to 14,
**characterized in that**
the protective sleeve (20) has at its upper cover region a grip element (25), at the underside (26) of which a ring element (27) with a formed-in 180°control cam (28) is formed in, which cooperates with a wedge (30) formed on the cover region (29) of the housing (4), which wedge prevents a turning back of the protective sleeve (20) after interlocking of the control cam (28) with the wedge (30).

16. The protective device according to Claims 11 to 14,
**characterized in that**
the grip element (25) is able to be fixed on the cover region (29) of the housing (4) by means of clamping tongues (33) engaging through the ring element (27).

## Revendications

1. Dispositif de protection (1) pour le tube d'aiguille (2) d'une seringue, comprenant
un boîtier à conformation pivotable (4) au niveau d'un élément porteur (3) et qui présente dans le plan de pivotement une face de boîtier ouverte (5), de sorte que le tube d'aiguille (2) est pivotable dans le boîtier (4) après usage pour injection,
l'élément porteur (3) et le boîtier pivotable (4) étant connectés en outre l'un à l'autre par une zone de rupture (9) près de la connexion d'une charnière en film (8) pour former un axe de pivotement,
**caractérisé en ce que**,
au niveau du boîtier, un dispositif de retenue (6) pour le tube d'aiguille pivoté vers l'intérieur (2) est prévu,
après usage pour injection, le boîtier (4) peut être repivoté vers l'arrière dans une position d'enclenchement au moyen de la zone de rupture détruite (9),
le tube d'aiguille (2) peut être amené en liaison fonctionnelle avec le dispositif de retenue (6) qui est prévu dans le boîtier (4).

2. Dispositif de retenue selon la revendication 1,
**caractérisé en ce que**
la zone de rupture (9) est composée d'éléments barrettes injectés restant souples (11).

3. Dispositif de retenue selon les revendications 1 et 2,
**caractérisé en ce que**
les éléments barrettes (11) sont disposés sur des zones périphériques (12, 13) de l'élément porteur (3) et du boîtier (4).

4. Dispositif de retenue selon les revendications 1 à 3,
**caractérisé en ce que**
les éléments barrettes (11) sont disposés répartis sur les zones périphériques (12, 13) de l'élément porteur (3) et du boîtier (4).

5. Dispositif de retenue selon les revendications 1 à 4,
**caractérisé en ce que**
le dispositif de retenue (6) est composé dans le boîtier (4) de deux languettes (14, 15) formées au niveau de la paroi du boîtier et qui sont orientées à la manière de harpons dans le sens de pivotement vers l'intérieur du tube d'aiguille (2).

6. Dispositif de retenue selon les revendications 1 à 5,
**caractérisé en ce que**,
entre les languettes (14, 15), il est prévu un élément tourillon (16) réalisé en forme triangulaire qui pousse le tube d'aiguille pivoté vers l'intérieur (2) sous l'une ou l'autre languette (14, 15).

7. Dispositif de retenue selon les revendications 1 à 6,
**caractérisé en ce que**
le dispositif de retenue (6) est composé d'un manchon (17) pouvant être enfoncé, qui est connecté par des zones de rupture en forme de dents de scie (18) au boîtier (4).

8. Dispositif de retenue selon la revendication 7,
**caractérisé en ce que**
les zones de rupture (18) sont formées au niveau des supports de barrettes (19) proéminents sur la face avant du boîtier.

9. Dispositif de retenue selon la revendication 8,
**caractérisé en ce que**
les supports de barrettes (19) sont formés au niveau de la paroi du boîtier.

10. Dispositif de retenue selon les revendications 1 à 9,
**caractérisé en ce que**
le dispositif de protection (1) est fabriqué avec l'élément porteur (3), le boîtier d'aiguille (4), la zone de rupture (9) et le dispositif de retenue (6) en une seule pièce à partir d'une pièce moulée par injection.

11. Dispositif de retenue selon les revendications 1 à 6,
**caractérisé en ce que**
le boîtier (4) s'appuyant de manière pivotable au niveau de l'élément porteur (3) est en option en liaison fonctionnelle avec un manchon de protection (20) au moyen duquel la face de boîtier ouverte (5) se trouvant dans le plan de pivotement peut être fermée après usage pour injection.

12. Dispositif de retenue selon la revendication 11,
**caractérisé en ce que**
le manchon de protection (20) présente une ouverture (21) pratiquée dans la paroi et qui est en recouvrement avec la face ouverte (5) se trouvant au niveau du boîtier.

13. Dispositif de retenue selon les revendications 11 et 12,
**caractérisé en ce que**
le manchon de protection (20) s'appuie en rotation au niveau du boîtier (4), de sorte que le manchon de protection (20) peut être tourné d'une position ouverte après usage pour injection vers une position fermant le boîtier (4).

14. Dispositif de retenue selon les revendications 11 à 13,
**caractérisé en ce que**
le manchon de protection (20) s'appuie en rotation par son bord inférieur de boîtier (22) au niveau de la zone périphérique (12) du boîtier (4), étant formé au niveau de la zone périphérique (12) du boîtier (4) un élément d'enclenchement (23) qui, après rotation du manchon de protection (20) de 180° au niveau du boîtier (4), vient en position d'enclenchement avec un (24) pratiqué dans la paroi du manchon de protection (20).

15. Dispositif de retenue selon les revendications 11 à 14,
**caractérisé en ce que**
le manchon de protection (20) présente, au niveau de sa zone de couverture supérieure, un élément de préhension (25) sur la face inférieure (26) duquel est formé un élément annulaire (27) avec une courbe de contrôle (28) de 180° formée à l'intérieur et qui est en liaison fonctionnelle avec une cale (30) formée sur la zone de couverture (29) du boîtier (4), laquelle cale empêche que le manchon protecteur (20) retourne vers l'arrière après enclenchement de la courbe de réglage (28) avec la cale (30).

16. Dispositif de retenue selon les revendications 11 à 14,
**caractérisé en ce que**
l'élément de préhension (25) peut être bloqué au moyen de languettes de serrage (33) passant à travers l'élément annulaire (27) au niveau de la zone de couverture (29) du boîtier (4).
